# EUROPÄISCHE PATENTSCHRIFT

(11) **EP 0 593 418 B1**
(45) Veröffentlichungstag und Bekanntmachung des Hinweises auf die Patenterteilung: **26.02.1997**
(21) Anmeldenummer: 89913157.7
(22) Anmeldetag: 24.11.1989
(51) Int. Cl.: C07D 487/04, A61K 31/505

(54) **ALKALI- UND ERDALKALISALZE DES OXIPURINOLS IN AMORPHER ODER KRISTALLINER FORM ALS MITTEL ZUR BEHANDLUNG VON HYPERURIKÄMIE UND GICHT**
ALKALI AND ALKALINE EARTH SALTS OF OXYPURINOL IN AMORPHOUS OR CRYSTALLINE FORM AS AGENTS FOR TREATING HYPERURICEMIA AND GOUT
UTILISATION DE SELS ALCALINS ET ALCALINO-TERREUX AMORPHES OU CRISTALLINS D'OXYPURINOL POUR LE TRAITEMENT DE L'HYPERURICEMIE ET DE LA GOUTTE

(30) Priorität: 25.11.1988 DE 3839826
(43) Veröffentlichungstag der Anmeldung: 27.04.1994
(73) Patentinhaber: Henning Berlin Anlagen GmbH, D-12099 Berlin (DE)
(72) Erfinder: SCHEIFFELE, Ekkehard, D-1000 Berlin 46 (DE); Weickgenannt, Guido, W-1000 Berlin 37 (DE)
(74) Vertreter: Werner, Hans-Karsten, Dr.Dipl.-Chem.
(86) Internationale Anmeldenummer: EP8901424
(87) Internationale Veröffentlichungsnummer: WO9006313

(56) Entgegenhaltungen:
- EP-A- 0 237 348
- GB-A- 975 850

## Beschreibung

Die Therapie der Hyperurikämie und Gicht kann mit Uricostatica oder Uricosurica oder mit der Kombination aus einem Uricostaticum und einem Uricosuricum durchgeführt werden. Als Uricostatica werden im allgemeinen die Xanthinoxidase-Hemmer Allopurinol bzw. Thiopurinol, als Uricosurica vor allem Sulfinpyrazon oder Benzbromaron verwendet. Untersuchungen über den Wirkungsmechanismus von Allopurinol haben ergeben, daß es selbst wenig zur erstrebten Wirkung beiträgt, sondern im wesentlichen erst nach Metabolisierung zu 4,6-Dihydroxy 3,4d pyrazolopyrimidin (Oxipurinol) seine therapeutische Wirkung entfaltet. Allopurinol und Oxipurinol hemmen das Enzym Xanthinoxidase gleich stark. Die Verweildauer von Allopurinol im Organismus - bei entsprechender galenischer Zubereitung nach oraler Gabe gut resorbierbar - beträgt jedoch nur etwa 6 Stunden; danach ist der Hauptteil in Oxipurinol umgewandelt, ein Teil wird zu Allopurinol-1-ribosid metabolisiert und weitere 3 bis 10% werden über die Niere ausgeschieden. Oxipurinol hat dagegen eine Halbwertzeit von ca. 22 Stunden und ist somit das eigentlich wirksame Prinzip einer Allopurinol-Therapie, während Allopurinol als Prodrug angesehen werden muß. Es wäre ein wesentlicher Fortschritt und von großem Vorteil, wenn es gelänge, Oxipurinol selbst zur Gicht-Therapie einsetzen zu können.

Es hat deshalb nicht an Versuchen gefehlt, statt des Prodrugs Allopurinol den eigentlichen Wirkstoff, also Oxipurinol, zu applizieren. Dies scheiterte jedoch daran, daß es nicht gelang, eine oral ausreichend resorbierbare galenische Zubereitung zu finden. So haben bereits Elion et al. (Renal Clearance of Oxipurinol, The Chief Metabolite of Allopurinol, American Journal of Medicine, Vol. 45, July 1968) solche Versuche, allerdings mit negativem Ergebnis, durchgeführt; vgl. auch Chalmers et al., Clin. Sci.(1968) 35, 353-362.

Auch durch den Einsatz von Oxipurinol in mikronisierter Form konnte keine praktikable Arzneiform geschaffen werden.

Aus der DE-OS 37 07 999 ist bekannt, Oxipurinol zur Verminderung von Zellschäden nach der Ischämie und vor der Reperfusion zu verwenden, und zwar durch intravenöse Verabreichung eines Alkalimetallsalzes, insbesondere des Natriumsalzes. Aus den Beispielen geht jedoch hervor, daß nicht das reine Natriumsalz verwendet wurde, sondern ein Gemisch aus Salz und überschüssiger Natronlauge. Dies ergibt sich vor allem aus den pH-Werten der ca. 1 bis 2%-igen Lösungen von 11,5 bzw. 12,0. Das jetzt erstmals von der Anmelderin in reiner und kristalliner Form hergestellte Natriumsalz besitzt in 5%-iger wäßriger Lösung einen pH-Wert von 9,7 und kristallisiert als Monohydrat aus.

Die Aufgabe, Oxipurinol wirksam zur Behandlung von Hyperurikämie und Gicht einzusetzen, konnte jetzt überraschend einfach dadurch gelöst werden, daß man Oxipurinol in seine Alkali- oder Erdalkalisalze überführt und diese in amorpher oder kristalliner Form oral appliziert.

Gegenstand der vorliegenden Erfindung sind somit Mittel zur oralen Behandlung der Hyperurikämie und Gicht enthaltend neben üblichen Träger- und Hilfsstoffen pharmakologisch wirksame Dosen von Alkali- und/oder Erdalkalisalzen des Oxipurinols in amorpher oder kristalliner Form. Vorzugsweise enthalten diese Mittel 50 bis 500 mg Wirkstoff pro Dosiseinheit.

Ein weiterer Gegenstand der Erfindung ist die Verwendung von Alkali- und/oder Erdalkalisalzen des Oxipurinols in amorpher oder kristalliner Form zur Herstellung von Mitteln zur oralen Behandlung der Hyperurikämie und Gicht.

Die Herstellung der Mittel zur oralen Behandlung der Hyperurikämie und Gicht erfolgt dadurch, daß man zunächst Oxipurinol in an sich bekannter Weise in seine Alkali- und Erdalkalisalze überführt und diese in amorpher oder kristalliner Form zusammen mit üblichen Träger- und Hilfsstoffen in oral applizierbare Arzneimittel füllt, beispielsweise Tabletten oder Kapseln.

Vergleichende Untersuchungen zwischen den erfindungsgemäßen Mitteln und Mitteln auf Basis von mikronisiertem Oxipurinol ergaben, daß die Resorptionsrate um den Faktor 3 erhöht wurde. Eine Untersuchung der Serumoxipurinolspiegel bei Applikation des erfindungsgemäßen Mittels im Vergleich zu Mitteln enthaltend Allopurinol ergab, daß die relative Resorptionsrate von Natrium-Oxipurinol im Vergleich zu Oxipurinol aus Allopurinol 87% beträgt. Eine vergleichende Untersuchung von Allopurinol und erfindungsgemäßen Mitteln enthaltend Natrium-Oxipurinol ergab, daß die Senkung des Serumharnsäurespiegels vergleichbar ist. Es ist somit möglich, anstatt Allopurinol die äquimolare Menge Natrium-Oxipurinol zu applizieren und dabei den gleichen therapeutischen Effekt zu erzielen, ohne den Körper durch die sonstigen Metaboliten des Allopurinols zu belasten.

Die Erfindung ist in den nachfolgenden Beispielen näher erläutert.

### Beispiel 1

### Natrium-Oxipurinol

In einer Mischung aus 25 1 reinem Methanol und 2,6 1 entionisiertem Wasser wird 1 kg trockenes Oxipurinol suspendiert und innerhalb 1 Stunde eine Lösung von 525 g NaOH, rein + 4,725 1 entionisiertem Wasser unter Rühren zugegeben. Es bildet sich ein Kristallbrei, der nach 3 Stunden Rührzeit abgetrennt (Nutsche bzw. Drucknutsche), portionsweise mit 10 1 reinem Methanol gewaschen und bei 60°C getrocknet wird.

Ausbeute ca. 95% Natrium-Oxipurinol.

### Eigenschaften von Natrium-Oxipurinol

Summenformel: C₅H₃N₄O₂Na · H₂O
MG. 192,1

Bei diesem Na-Salz handelt es sich um Mono-Natriumoxipurinol-Monohydrat. Das Salz ist weiß und kristallisiert in feinen Nadeln. In wäßriger Lösung reagiert es alkalisch, eine 5%-ige Lösung besitzt einen pH-Wert von 9,7. Seine Löslichkeit beträgt:

| | |
|---|---|
| in Wasser bei 25° | 15,8 g/l |
| 0° | 7,2 g/l |

in Methanol/Wasser (3:1 Vol.) bei 25° ca. 2,0 g/l

Das Salz ist luftbeständig und verträgt Trockentemperaturen bis etwa 70°. Aus seiner heißen Lösung läßt sich mit verdünnter Salzsäure (10%-ig) Oxipurinol fast quantitativ ausfällen.

### Beispiel 2

### Kalium-Oxipurinol

Wie in Beispiel 1 wird 1 kg Oxipurinol in 90% Methanol suspendiert und entsprechend mit 735 g KOH umgesetzt.

### Ausbeute ca. 85% Kalium-Oxipurinol

### Beispiel 3

### Magnesium-Oxipurinol

Die wäßrige Aufschlämmung von reinem Oxipurinol (2 Mol) mit 1 Mol Magnesium-Chlorid wird bei ca. 60° unter Rühren mit einem Überschuß an stark basischem Ionenaustauscherharz (z.B. Amberlite IRA 400 oder DOWEX 1 SBR) umgesetzt, das Harz abgetrent und nach Einengen das Magnesiumsalz mit Isopropanol ausgefällt. Das Magnesiumsalz fällt hierbei amorph an.

### Beispiel 4

### Natrium-Oxipurinol-Tabletten

113,7 g Natrium-Oxipurinol gemäß Beispiel 1 werden mit 30 g mikrokristalliner Cellulose, 6,0 g quervernetztem Polyvinylpyrrolidon sowie 2,1 g Polyvinylpyrrolidon (mittl. Mol.-Gew. ca. 25000) trocken vermischt, mit Wasser angefeuchtet, das entstandene Granulat getrocknet und anschließend mit 1,2 g Magnesiumstearat vermischt. Aus dem Granulat werden in üblicher Weise Tabletten mit ca. 60 bis 500 mg Wirkstoff gepreßt, diese können mit einem Film aus Hydroxypropylmethylcellulose überzogen werden. Es können auch Mini-Filmtabletten hergestellt und in Hartgelatinekapseln abgefüllt werden.

### Beispiel 5

### Kalium-Oxipurinol-Tabletten

Unter Verwendung von Kalium-Oxipurinol oder Magnesium-Oxipurinol statt des Natriumsalzes wird ein Granulat gemäß Beispiel 4 hergestellt. Daraus lassen sich Tabletten in therapiegerechter Dosis, vorzugsweise 60 bis 600 mg pro Tablette, pressen.

### Beispiel 6

### Oxipurinol im Serum nach oraler Applikation von Natrium-Oxipurinol-Tabletten bzw. Tabletten mit mikronisiertem Oxipurinol

6 Probanden erhielten in einem einfachen Cross over design je 1 Tablette mit 384 mg Natrium-Oxipurinol bzw. 336 mg Oxipurinol mikronisiert. Blutproben wurden zu den üblichen engmaschigen Zeitpunkten abgenommen und die Oxipurinol-Konzentrationen im Serum bestimmt. Aus der Fläche unter der Serumverlaufskurve wurde die relative Resorptionsrate errechnet. Sie betrug für das mikronisierte Oxipurinol-Präparat 33% von der für Natrium-Oxipurinol.

### Beispiel 7

### Oxipurinol im Serum nach oraler Applikation von Allopurinol bzw. Natrium-Oxipurinol-Tabletten

Wie in Beispiel 6 wurde der Verlauf der Serum-Oxipurinol-Spiegel und die Flächen unter den Kurven nach Gabe von 384 mg Natrium-Oxipurinol bzw. 300 mg Allopurinol (Standard-Gicht-Therapeutikum) bestimmt. Die relative Resorptionsrate von Natrium-Oxipurinol im Vergleich zu Oxipurinol aus Allopurinol betrug 87%.

### Beispiel 8

### Harnsäure-senkende Wirkung von Natrium-Oxipurinol-Tabletten

Je 10 Patienten mit wäßriger Hyperurikämie (Serum-Harnsäure 7,9 ± 1,3 bzw. 7,8 ± 1,2mg/100 ml) erhielten über einen Zeitraum von zwei Wochen täglich 384 mg Natrium-Oxipurinol bzw. 300 mg Allopurinol. In beiden Kollektiven kam es zu einer etwa gleich starken Senkung des Serum-Harnsäurespiegels um 2,1 bzw. 2,0 mg/100 ml.

## Patentansprüche

1. Mittel zur oralen Behandlung der Hyperurikämie und Gicht enthaltend neben üblichen Träger- und Hilfsstoffen pharmakologisch wirksame Dosen von Alkali- und/oder Erdalkalisalzen des Oxipurinols in amorpher oder kristalliner Form.

2. Mittel gemäß Anspruch 1 enthaltend 50 bis 500 mg Wirkstoff pro Dosiseinheit.

3. Verwendung von Alkali- und/oder Erdalkalisalzen des Oxipurinols in amorpher oder kristalliner Form zur Herstellung von Mitteln zur oralen Behandlung der Hyperurikämie und Gicht.

## Claims

1. Agents for oral treatment of hyperuricemia and gout, containing pharmacologically active amounts of alkali metal and/or alkaline earth metal salts of oxypurinol in amorphous or crystalline form, in addition to conventional vehicles and additives.

2. Agents according to claim 1, containing from 50 to 500 mg of active ingredient per unit dose.

3. Use of alkali metal and/or alkaline earth metal salts of oxypurinol in amorphous or crystalline form for the preparation of agents for oral treatment of hyperuricemia and gout.

## Revendications

1. Agent pour le traitement oral de l'hyperuricémie et de la goutte, contenant, en plus des véhicules et adjuvants usuels, des doses pharmacologiquement efficaces de sels alcalins et/ou alcalino-terreux d'oxypurinol, à l'état amorphe ou cristallin.

2. Agent selon la revendication 1, contenant de 50 à 500 mg de principe actif par dose unitaire.

3. Utilisation de sels alcalins et/ou alcalino-terreux d'oxypurinol, à l'état amorphe ou cristallin, pour la préparation d'agents destinés au traitement oral de l'hyperuricémie et de la goutte.
